# FASCICULE DE BREVET EUROPEEN

(11) **EP 0 972 042 B1**
(45) Date de publication et mention de la délivrance du brevet: **12.04.2006**
(21) Numéro de dépôt: 98920561.2
(22) Date de dépôt: 27.03.1998
(51) Int. Cl.: C12N 15/12, C07K 14/47, A61K 38/17, C07K 16/18

(54) **POLYPEPTIDE EXPRIMEE DANS LA COUCHE CORNEE DE L'EPIDERME ET SON UTILISATION**
EPIDERMALES PROTEIN AUS DER HORNHAUTSCHICHT UND SEINE VERWENDUNG
POLYPEPTIDE EXPRESSED IN THE HORNY LAYER OF EPIDERMIS AND USE THEREOF

(30) Priorité: 28.03.1997 FR 9703899; 11.09.1997 FR 9711317
(43) Date de publication de la demande: 19.01.2000
(73) Titulaire: L'OREAL, 75008 Paris (FR)
(72) Inventeur: SERRE, Guy, Bruno, René, 10, avenue W. Churchill F-31100 Toulouse (FR); SIMON, Michel, F-31450 Belberaud (FR); WEBER-VIVAT, Marina, F-31290 Villefranche de Lauragais (FR)
(74) Mandataire: Allab, Myriam
(86) Numéro de dépôt international: PCT/FR1998/000636
(87) Numéro de publication internationale: WO 1998/044105

(56) Documents cités:
- ZHOU Y. ET AL: "Identification in the HLA class I region of a gene expressed late in keratinocyte differentiation" PROCEEDINGS OF THE NATIONAL ACADEMY OF SCIENCES OF USA., vol. 90, 1993, pages 9470-9474, XP002049396 WASHINGTON US
- LUNDSTROM A ET AL: "Evidence for a role of corneodesmosin, a protein which may serve to modify desmosomes during cornification, in stratum corneum cell cohesion and desquamation" ARCHIVES OF DERMATOLOGICAL RESEARCH, 286 (7). 1994. 369-375., XP002075787
- SIMON M ET AL: "Characterization and purification of human corneodesmosin, an epidermal basic glycoprotein associated with corneocyte-specific modified desmosomes" JOURNAL OF BIOLOGICAL CHEMISTRY, 272 (50). 1997. 31770-31776., XP002075788
- SERRE G ET AL.: "Identification of late differentiation antigens of human cornified epithelia, expressed in re-organized desmosomes and bound to cross-linked envelope" JOURNAL INVEST. DERMATOL., vol. 103, 1994, pages 731-740, XP002075789

## Description

L'invention a pour objet une composition cosmétique ou pharmaceutique comprenant, dans un milieu physiologiquement acceptable, au moins un polypeptide naturel ou synthétique purifié, spécifique de l'épiderme, ayant un rôle dans la cohésion intercornéocytaire. L'invention a également pour objet une composition cosmétique ou pharmaceutique comprenant un mélange de polypeptides issus de la protéolyse du polypeptide purifié, un procédé de traitement cosmétique destiné à renforcer la cohésion intercornéocytaire et un procédé de traitement cosmétique destiné à diminuer la cohésion intercornéocytaire, donc à favoriser la desquamation.

La peau humaine est constituée de deux compartiments à savoir un compartiment profond, le derme, et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également constitué de vaisseaux sanguins et de fibres nerveuses.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

L'épiderme est conventionnellement divisé en une couche basale de kératinocytes qui constitue la couche germinative de l'épiderme, une couche dite épineuse constituée de plusieurs couches de cellules polyédriques disposées sur les cellules germinatives, une couche dite granuleuse constituée de cellules aplaties contenant des inclusions cytoplasmiques distinctes, les grains de kératohyaline, et enfin une couche supérieure appelée couche cornée (ou stratum corneum), constituée de kératinocytes au stade terminal de leur différenciation appelés cornéocytes. Ceux-ci sont des cellules momifiées, anucléées qui dérivent des kératinocytes.

Les cornéocytes sont principalement composés d'une matrice fibreuse contenant des cytokératines, entourée d'une structure très résistante de 15 nm d'épaisseur, appelée enveloppe cornée ou cornifiée. L'empilement de ces cornéocytes constitue la couche cornée qui est responsable de la fonction de barrière de l'épiderme. Au cours du processus normal de desquamation, les cornéocytes les plus superficiels se détachent de la surface de l'épiderme.
Des structures intercellulaires dérivant des desmosomes, appelées cornéosomes ou cornéodesmosomes, ont été décrites dans la couche cornée. Des études récentes ont montré leur importance majeure dans la cohésion intercornéocytaire ainsi que dans le processus de desquamation. En particulier, une corrélation étroite existe entre la dissociation cellulaire et la protéolyse de certains composants cornéodesmosomaux comme la desmogléine I.
Plusieurs protéases à sérine de type trypsine ou chymotrypsine semblent être impliquées dans la protéolyse des cornéodesmosomes, en particulier l'enzyme chymotrypsique de la couche cornée (stratum corneum chymotryptic enzyme).

De nombreuses pathologies cutanées se caractérisent par la production d'une couche cornée épaissie et par une desquamation anormale, c'est-à-dire par une hyperkératose. Celle-ci peut survenir sur tout territoire anatomique cutané et dans des contextes cliniques très variés. Son substratum physiopathologique et sa cause sont variés.

A titre d'exemples on peut citer :
- la xérose (ou sécheresse cutanée),
- les ichthyoses,
- le psoriasis,
- certaines lésions tumorales bénignes ou malignes,
- les hyperkératoses réactionnelles.

D'autres pathologies se caractérisent par une transdifférenciation ou métaplasie, au niveau de muqueuses, malpighiennes ou non, mais normalement non cornifiées, qui deviennent cornifiées, c'est-à-dire se revêtent d'un épithélium anormal, producteur à sa surface d'une couche cornée. Bien que les muqueuses génitales et celles des voies aérodigestives supérieures soient le plus souvent concernées, ces métaplasies peuvent siéger dans divers territoires anatomiques.

A titre d'exemples on peut citer :
- la leukokératose du col utérin au cours du prolapsus,
- les leukokératoses buccales,
- les lésions tumorales bénignes kératosiques des muqueuses malpighiennes.

A l'inverse, certaines manifestations pathologiques entraînent un amincissement de l'épiderme et en particulier de la couche cornée, se traduisant par une fragilité excessive du revêtement cutané. Celle-ci peut siéger dans divers territoires anatomiques, sa cause est variable et elle peut être constitutionnelle ou acquise.

A titre d'exemples on peut citer :
- les troubles trophiques cutanés des membres inférieurs chez les patients porteurs de pathologies vasculaires : varices, artériopathies (diabète, artériosclérose...),
- les troubles trophiques cutanés dans le cadre d'un syndrome algodystrophique,
- les troubles trophiques consécutifs à une cicatrisation anormale.

La purification et la connaissance des polypeptides impliqués dans la cohésion intercornéocytaire est une des voies qui pourrait permettre l'élaboration de produits destinés à lutter contre les effets d'un excès ou d'un défaut en polypeptides de ce type en particulier à la surface de la peau.

L'article du Journal Invest. Dermatol. (**97**, 1991, pages 1061-1072), rapporte l'électrophorèse sur gel SDS-PAGE d'un mélange de protéines extraites du stratum corneum. Les antigènes sont mis en évidence par marquage par des anticorps murins et on leur attribue des poids moléculaire apparents de 33,5 à 52 kD, en fonction de leur migration sur le gel. Aucun isolement subséquent de ces protéines n'est réalisé:

Lundström et al (Arch. Dermatol. Res. **286**, 1994, pages 369-375) ont étudié le rôle de la cornéodesmosine dans la cohésion cellulaire et suggèrent l'existence de protéines de poids moléculaire apparent de 33 à 48 kDa, sans toutefois les isoler. ZHOU et al. (P.N.A.S., vol.90, 1993) concerne l'identification dans la région HLA de classe I d'un gène exprimé tardivement dans la différenciation du kératinocyte. Ce document rapporte les résultats des expériences ayant conduit à l'identification de la séquence du gène S.

Un des objets de l'invention est de fournir une composition comprenant un polypeptide impliqué dans la cohésion intercornéocytaire sous forme purifiée.

Après de longs et laborieux travaux du fait de sa faible représentation parmi les protéines de l'épiderme, de sa grande instabilité et de sa sensibilité élevée aux protéases, la demanderesse a mis en évidence, isolé et purifié par des techniques biochimiques à partir d'épiderme humain, un polypeptide spécifique des épithéliums cornifiés. Ce polypeptide, que l'on nommera également par ailleurs dans le texté "cornéodesmosine" est exprimé dans la couche cornée de l'épiderme et est impliqué dans la cohésion intercornéocytaire. La demanderesse en a déterminé la séquence primaire en acides aminés.

L'invention a donc pour objet une composition cosmétique ou pharmaceutique comprenant, dans un milieu physiologiquement acceptable, au moins un polypeptide naturel ou synthétique purifié, ledit polypeptide étant caractérisé par le fait qu'il répond à la séquence d'acides aminés SEQ ID NO : 1 suivante :

Met Gly Ser Ser Arg Ala Pro Trp Met Gly Arg Val Gly Gly His Gly
1               5                   10                  15
Met Met Ala Leu Leu Leu Ala Gly Leu Leu Leu Pro Gly Thr Leu Ala
                20                  25                  30
Lys Ser Ile Gly Thr Phe Ser Asp Pro Cys Lys Asp Pro Thr Arg Ile
            35                  40                  45
Thr Ser Pro Asn Asp Pro Cys Leu Thr Gly Lys Gly Asp Ser Ser Gly
    50                  55                  60
Phe Ser Ser Tyr Ser Gly Ser Ser Ser Ser Gly Ser Ser Ile Ser Ser
65                  70                  75                  80
Ala Arg Ser Ser Gly Gly Gly Ser Ser Gly Ser Ser Ser Gly Ser Ser
                85                  90                  95
Ile Ala Gln Gly Gly Ser Ala Gly Ser Phe Lys Pro Gly Thr Gly Tyr
            100                 105                 110
Ser Gln Val Ser Tyr Ser Ser Gly Ser Gly Ser Ser Leu Gln Gly Ala
        115                 120                 125
Ser Gly Ser Ser Gln Leu Gly Ser Ser Ser Ser His Ser Gly Ser Ser
    130                 135                 140
Gly Ser His Ser Gly Ser Ser Ser Ser His Ser Ser Ser Ser Ser Ser
145                 150                 155                 160
Phe Gln Phe Ser Ser Ser Ser Phe Gln Val Gly Asn Gly Ser Ala Leu
                165                 170                 175
Pro Thr Asn Asp Asn Ser Tyr Arg Gly Ile Leu Asn Pro Ser Gln Pro
            180                 185                 190
Gly Gln Ser Ser Ser Ser Ser Gln Thr Ser Gly Val Ser Ser Ser Gly
        195                 200                 205
Gln Ser Val Ser Ser Asn Gln Arg Pro Cys Ser Ser Asp Ile Pro Asp
    210                 215                 220
Ser Pro Cys Ser Gly Gly Pro Ile Val Ser His Ser Gly Pro Tyr Ile
225                 230                 235                 240
Pro Ser Ser His Ser Val Ser Gly Gly Gln Arg Pro Val Val Val Val
                245                 250                 255
Val Asp Gln His Gly Ser Gly Ala Pro Gly Val Val Gln Gly Pro Pro

            260                 265                 270
Cys Ser Asn Gly Gly Leu Pro Gly Lys Pro Cys Pro Pro Ile Thr Ser
        275                 280                 285
Val Asp Lys Ser Tyr Gly Gly Tyr Glu Val Val Gly Gly Ser Ser Asp
    290                 295                 300
Ser Tyr Leu Val Pro Gly Met Thr Tyr Ser Lys Gly Lys Ile Tyr Pro
305                 310                 315                 320
Val Gly Tyr Phe Thr Lys Glu Asn Pro Val Lys Gly Ser Pro Gly Val
                325                 330                 335
Pro Ser Phe Ala Ala Gly Pro Pro Ile Ser Glu Gly Lys Tyr Phe Ser
            340                 345                 350
Ser Asn Pro Ile Ile Pro Ser Gln Ser Ala Ala Ser Ser Ala Ile Ala
        355                 360                 365
Phe Gln Pro Val Gly Thr Gly Gly Val Gln Leu Cys Gly Gly Gly Ser
    370                 375                 380
Thr Gly Ser Lys Gly Pro Cys Ser Pro Ser Ser Ser Arg Val Pro Ser
385                 390                 395                 400
Ser Ser Ser Ile Ser Ser Ser Ala Gly Ser Pro Tyr His Pro Cys Gly
                405                 410                 415
Ser Ala Ser Gln Ser Pro Cys Ser Pro Pro Gly Thr Gly Ser Phe Ser
            420                 425                 430
Ser Ser Ser Ser Ser Gln Ser Ser Gly Lys Ile Ile Leu Gln Pro Cys
        435                 440                 445
Gly Ser Lys Ser Ser Ser Ser Gly His Pro Cys Met Ser Val Ser Ser
    450                 455                 460
Leu Thr Leu Thr Gly Gly Pro Asp Gly Ser Pro His Pro Asp Pro Ser
465                 470                 475                 480
Ala Gly Ala Lys Pro Cys Gly Ser Ser Ser Ala Gly Lys Ile Pro Cys
                485                 490                 495
Arg Ser Ile Arg Asp Ile Leu Ala Gln Val Lys Pro Leu Gly Pro Gln
            500                 505                 510
Leu Ala Asp Pro Glu Val Phe Leu Pro Gln Gly Glu Leu Leu Asp Ser
        515                 520                 525
Pro

Le polypeptide de l'invention peut être d'origine naturelle ou synthétique. Par synthétique on entend ici tout polypeptide obtenu chimiquement ou par production dans un organisme après introduction dans cet organisme des éléments nécessaires à cette production.

Le polypeptide de l'invention peut être issu de toute origine possible à savoir soit animale, en particulier de mammifères et encore plus particulièrement humaine, soit végétale, soit de micro-organismes (virus, phages, bactéries entre autres) ou encore de champignons, sans préjuger du fait qu'il soit présent de manière naturelle ou non dans ledit organisme d'origine.

Préférentiellement, le polypeptide de l'invention est d'origine naturelle, purifié à partir de tissus de mammifères, particulièrement à partir de peau de mammifères.

Préférentiellement, le polypeptide de l'invention est purifié à partir de peau humaine et encore plus préférentiellement à partir d'épiderme humain.

Comme indiqué précédemment, la cohésion intercornéocytaire est apparemment due entre autres à l'existence dans la couche cornée de polypeptides spécifiques des structures impliquées dans la jonction intercornéocytaire.

Ainsi, le polypeptide de l'invention est spécifique de la couche cornée et de la couche granuleuse et, préférentiellement, le polypeptide selon l'invention est spécifique des structures impliquées dans la jonction intercornéocytaire, particulièrement des cornéodesmosomes.

On sait qu'en général les polypeptides matures que l'on trouve dans les cellules proviennent de la maturation de précurseurs qui contiennent dans leur séquence la séquence du polypeptide mature.

Ainsi, l'invention concerne également une composition cosmétique ou pharmaceutique comprenant, dans un milieu physiologiquement acceptable, tout polypeptide dont la séquence est en partie constituée par la séquence du polypeptide de l'invention.

Il est connu également que les polypeptides peuvent subir des modifications post-traductionnelies comme la formation de liaisons disulfure, les clivages protéolytiques spécifiques, l'addition de glucides (glycosylation), la phosphorylation en particulier au niveau des sérines et/ou des thréonines et/ou des tyrosines, et/ou l'association à des lipides.

L'invention concerne donc une composition cosmétique ou pharmaceutique comprenant, dans un milieu physiologiquement acceptable, au moins un polypeptide de l'invention ayant subi ou non des modifications post-traductionnelles.

Le polypeptide de l'invention peut avoir subi une ou plusieurs modifications post-traductionnelles.

Préférentiellement, le polypeptide selon l'invention est glycosylé et/ou phosphorylé.

On sait classer les polypeptides en fonction de leur point isoélectrique. Préférentiellement, le polypeptide de l'invention est basique.

Bien entendu la séquence primaire en acides aminés, ainsi que les diverses modifications post-traductionnelles subies par le polypeptide font que ledit polypeptide peut être caractérisé par son poids moléculaire, exprimé en kilodaltons.
Le polypeptide de l'invention a un poids moléculaire apparent déterminé par électrophorèse en gel de polyacrylamide SDS (PAGE-SDS) compris entre 50 et 60 kilodaltons, préférentiellement, entre 52 et 56 kilodaltons.

Très préférentiellement, le polypeptide de l'invention est un polypeptide glycosylé,
phosphorylé, basique d'un poids moléculaire apparent compris entre 50 et 60, de préférence entre 52 et 56, kilodaltons.

Ainsi préférentiellement, l'invention a pour objet une composition cosmétique ou pharmaceutique comprenant, dans un milieu physiologiquement acceptable, au moins un polypeptide de l'invention, ledit polypeptide étant glycosylé, basique, phosphorylé et ayant un poids moléculaire apparent compris entre 50 et 60 kilodaltons, préférentiellement entre 52 et 56 kilodaitons.

Il est par ailleurs connu que la séquence primaire en acides aminés d'un polypeptide détermine des sites spécifiquement reconnus par des protéases qui une fois la reconnaissance de ces sites effective vont, avec ou sans fixation audit polypeptide, induire son clivage par protéolyse.

Ainsi, l'invention concerne également une composition cosmétique ou pharmaceutique comprenant, dans un milieu physiologiquement acceptable, au moins un mélange de polypeptides issus de la protéolyse du polypeptide de l'invention.

Comme décrit précédemment certaines pathologies peuvent être dues à un excès de desquamation dont on peut supposer qu'il est dû à un défaut en polypeptides impliqués dans la cohésion intercornéocytaire.

Certaines manifestations pathologiques entraînent un amincissement de l'épiderme et en particulier de la couche cornée, se traduisant par une fragilité excessive du revêtement cutané, qui peut siéger dans tout territoire anatomique cutané, être de cause variée constitutionnelle ou acquise.

Ainsi, la composition selon l'invention est destinée à traiter l'amincissement de l'épiderme, et en particulier de la couche cornée, et/ou à traiter une fragilité excessive du revêtement cutané et/ou à renforcer la cohésion intercornéocytaire et/ou induire l'épaississement de la couche cornée.

De même, la composition selon l'invention est destinée à traiter l'amincissement de l'épiderme, et en particulier de la couche cornée, et/ou à traiter une fragilité excessive du revêtement cutané et/ou à renforcer la cohésion intercornéocytaire et/ou induire l'épaississement de la couche cornée.

De la même façon, la composition de l'invention peut être utilisée pour provoquer un épaississement localisé de la couche cornée au niveau de territoires cutanés devant subir des microtraumatismes répétés.

A titre d'exemple on peut citer le traitement préventif des bulles sous-épidermiques ("ampoules") chez les sportifs.

L'invention a également pour objet un procédé de traitement cosmétique destiné à traiter l'amincissement de l'épiderme, et en particulier de la couche cornée, et/ou à traiter une fragilité excessive du revêtement cutané et/ou à renforcer la cohésion intercornéocytaire et/ou induire l'épaississement de la couche cornée, caractérisé par le fait que l'on applique sur la peau du sujet à traiter une composition cosmétique selon l'invention

L'invention a également pour objet un procédé de traitement cosmétique destiné à traiter les troubles trophiques cutanés, par exemple des patients porteurs de pathologies vasculaires telles que les varices ou les artériopathies (diabète, artériosclérose...), les troubles trophiques cutanés des patients porteurs de syndromes algo-dystrophiques ou ceux consécutifs à des troubles de la cicatrisation.

L'analyse dé la séquence primaire en acides aminés de la protéine selon l'invention montre qu'elle présente des sites de reconnaissance et de fixation pour des protéases connues ou des sites spécifiques de clivage par des agents chimiques. On peut citer comme exemple les sites pour la Chymotrypsine, la Cathépsine, la protéinase K, la Subtilisine, la protéase V8, la Thermolysine, la Thrombine, la Trypsine, la Papaïne, la Pepsine, la Proline-Endopeptidase, l'Endoprotéinase GluC, l'Endoprotéinase LysC, l'Endoprotéinase AspN, l'Endoprotéinase ArgC (Clostripaïne), la Myxobacter AL117, l'Elastase, l'enzyme chymotrypsique de la couche cornée, le bromure de cyanogène, le N-chlorosuccinimide ou encore les sites spécifiques de l'hydrolyse acide (70% d'acide formique, 7M Guanidine-HCI, 40°C, 24h).

La cohésion intercornéocytaire est apparemment due à l'existence, dans la couche cornée, de polypeptides spécifiques des structures impliquées dans la jonction intercornéocytaire comme en particulier le polypeptide de l'invention. On a vu que certaines pathologies hyperkératosiques pourraient être liées à un excès de cohésion intercornéocytaire, dû en particulier au polypeptide de l'invention.

Ces protéases peuvent être isolées à partir de plantes, d'animaux, de bactéries, de virus ou de champignons.

Le polypeptide de l'invention peut être glycolysé. Ainsi, la composition telle que définie précédemment peut contenir en outre une glycosidase qui peut être choisie parmi celles isolées de plantes, d'animaux, de champignon ou encore de micro-organismes, en particulier de bactéries. On citera à titre d'exemple les neuraminidases, les mannosidases, les galactosidases, les glucosidases, les N-acétylglucosaminidases et les N-acétylgalactosaminidases.

Comme vu précédemment certaines pathologies se caractérisent par la production d'une couche cornée épidermique épaissie et par une desquamation anormale, c'est-à-dire par une hyperkératose qui peut survenir dans tout territoire anatomique cutané, dans divers contextes cliniques et dont le substratum physiopathologique et la cause peuvent être variés.

On sait qu'une protéine est synthétisée dans les cellules à partir d'une matrice d'acides désoxyribonucléiques codant pour ladite protéine. On sait également que le code génétique est dégénéré. Ainsi, la séquence d'acides aminés du polypeptide de l'invention peut être issue de différentes séquences d'acides désoxyribonucléiques, naturelles ou synthétiques. Par séquence d'acides désoxyribonucléiques synthétique, on entend ici toute séquence obtenue chimiquement ou par manipulation génétique.

Lesdites séquences d'acides désoxyribonucléiques peuvent être issues de toutes origines possibles à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentés de manière naturelle ou non dans ledit organisme d'origine.

La demanderesse a isolé, purifié et séquencé par des techniques de biologie moléculaire en particulier le criblage de banques d'expression d'acides désoxyribonucléiques complémentaires préparées à partir d'épiderme humain, un fragment d'acides désoxyribonucléiques codant pour le polypeptide de l'invention.

L'invention a donc également pour objet une composition cosmétique ou pharmaceutique comprenant, dans un milieu physiologiquement acceptable, toutes séquences d'acides désoxyribonucléiques, naturelles ou synthétiques, dont tout ou partie code pour la séquence primaire d'acides aminés du polypeptide de l'invention.

Au cours de ces travaux, la demanderesse a pu isoler et purifier une séquence d'acides désoxyribonucléiques codant pour la séquence primaire d'acides aminés du polypeptide de l'invention à partir de peau humaine.

Particulièrement l'invention a pour objet une composition cosmétique ou pharmaceutique comprenant, dans un milieu physiologiquement acceptable, un fragment isolé d'acide désoxyribonucléique comprenant au moins la séquence nucléotidique codante SEQ ID NO : 2 suivante :

1 ATG GGC TCG TCT CGG GCA CCC TGG ATG GGG CGT GTG GGT GGG CAC
46 GGG ATG ATG GCA CTG CTG CTG GCT GGT CTC CTC CTG CCA GGG ACC
91 TTG GCT AAG AGC ATT GGC ACC TTC TCA GAC CCC TGT AAG GAC CCC
136 ACG CGT ATC ACC TCC CCT AAC GAC CCC TGC CTC ACT GGG AAG GGT
181 GAC TCC AGC GGC TTC AGT AGC TAC AGT GGC TCC AGC AGT TCT GGC
226 AGC TCC ATT TCC AGT GCC AGA AGC TCT GGT GGT GGC TCC AGT GGT
271 AGC TCC AGC GGA TCC AGC ATT GCC CAG GGT GGT TCT GCA GGA TCT
316 TTT AAG CCA GGA ACG GGG TAT TCC CAG GTC AGC TAC TCC TCC GGA
361 TCT GGC TCT AGT CTA CAA GGT GCA TCC GGT TCC TCC CAG CTG GGG
406 AGC AGC AGC TCT CAC TCG GGA AGC AGC GGC TCT CAC TCG GGA AGC
451 AGC AGC TCT CAT TCG AGC AGC AGC AGC AGC TTT CAG TTC AGC AGC
496 AGC AGC TTC CAA GTA GGG AAT GGC TCT GCT CTG CCA ACC AAT GAC
541 AAC TCT TAC CGC GGA ATA CTA AAC CCT TCC CAG CCT GGA CAA AGC
586 TCT TCC TCT TCC CAA ACC TCT GGG GTA TCC AGC AGT GGC CAA AGC
631 GTC AGC TCC AAC CAG CGT CCC TGT AGT TCG GAC ATC CCC GAC TCT
676 CCC TGC AGT GGA GGG CCC ATC GTC TCG CAC TCT GGC CCC TAC ATC
721 CCC AGC TCC CAC TCT GTG TCA GGG GGT CAG AGG CCT GTG GTG GTG
766 GTG GTG GAC CAG CAC GGT TCT GGT GCC CCT GGA GTG GTT CAA GGT
811 CCC CCC TGT AGC AAT GGT GGC CTT CCA GGC AAG CCC TGT CCC CCA
856 ATC ACC TCT GTA GAC AAA TCC TAT GGT GGC TAC GAG GTG GTG GGT
901 GGC TCC TCT GAC AGT TAT CTG GTT CCA GGC ATG ACC TAC AGT AAG
946 GGT AAA ATC TAT CCT GTG GGC TAC TTC ACC AAA GAG AAC CCT GTG
991 AAA GGC TCT CCA GGG GTC CCT TCC TTT GCA GCT GGG CCC CCC ATC
1036 TCT GAG GGC AAA TAC TTC TCC AGC AAC CCC ATC ATC CCC AGC CAG
1081 TCG GCA GCT TCC TCG GCC ATT GCG TTC CAG CCA GTG GGG ACT GGT
1126 GGG GTC CAG CTC TGT GGA GGC GGC TCC ACG GGC TCC AAG GGA CCC
1171 TGC TCT CCC TCC AGT TCT CGA GTC CCC AGC AGT TCT AGC ATT TCC
1216 AGC AGC GCC GGT TCA CCC TAC CAT CCC TGC GGC AGT GCT TCC CAG
1261 AGC CCC TGC TCC CCA CCA GGC ACC GGC TCC TTC AGC AGC AGC TCC
1306 AGT TCC CAA TCG AGT GGC AAA ATC ATC CTT CAG CCT TGT GGC AGC
1351 AAG TCC AGC TCT TCT GGT CAC CCT TGC ATG TCT GTC TCC TCC TTG
1396 ACA CTG ACT GGG GGC CCC GAT GGC TCT CCC CAT CCT GAT CCC TCC
1441 GCT GGT GCC AAG CCC TGT GGC TCC AGC AGT GCT GGA AAG ATC CCC
1486 TGC CGC TCC ATC CGG GAT ATC CTA GCC CAA GTG AAG CCT CTG GGG
1531 CCC CAG CTA GCT GAC CCT GAA GTT TTC CTA CCC CAA GGA GAG TTA
1576 CTC GAC AGT CCA TAA

Quelle que soit leur nature, les compositions de l'invention peuvent être ingérées, injectées ou appliquées sur la peau (sur toute zone cutanée du corps) ou les muqueuses (buccale, jugale, gingivale, génitale, conjonctivale, ...).

De préférence, les compositions de l'invention sont appliquées sur la peau ou les muqueuses.

Selon le mode d'administration, les compositions selon l'invention peuvent se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses ou encore sous forme de compositions pour aérosol ou de mousses ou encore sous forme de compositions pour aérosol comprenant également un agent propulseur sous pression. Ces compositions sont préparées selon les méthodes usuelles.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour les soins du cuir chevelu, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.
Comme gélifiants hydrophiles utilisables dans l'invention, on peut citer les polymères carboxyvinyliques (carbomer), les copolymères acryliques tels que les copolymères d'acrylateslalkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéines, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait d'au moins une Iridacée à d'autres agents actifs destinés notamment à la prévention et/ou au traitement des affections cutanées. Parmi ces agents actifs, on peut citer à titre d'exemple :
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, selon un mode particulier, la composition selon l'invention comprend également au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents diminuant la différenciation et/ou la prolifération et/ou la pigmentation cutanée.

### EXEMPLES :

### Anticorps monoclonaux :

Les anticorps monoclonaux murins G36-19, F28-27 et B17-21 (IgG1) spécifiques du polypeptide de l'invention, la cornéodesmosine, font partie d'une série d'anticorps dirigés contre des antigènes de différenciation épidermique, produits après immunisation d'une souris avec un homogénat de couche cornée plantaire humaine, puis caractérisés. L'ascite de l'anticorps monoclonal MOPC-21 (IgG1) (Sigma Chemical Co., St Louis, MO) a été utilisée comme contrôle négatif. Le kit d'anticorps anti-phosphosérine de Biomol Feinchenukalien GmbH (Anticorps monoclonal 1 C8, 4A3, 4A9 et 4H4) et l'anticorps monoclonal PSR45 de Sigma ont aussi été utilisés.

### Isolement et caractérisation du polypeptide :

### Extraction protéique séquentielle :

A partir de peau mammaire (provenant de plasties mammaires de réduction), l'épiderme a été séparé du derme mécaniquement après traitement à la chaleur de la peau pendant 5 minutes à 56°C dans un tampon phosphate, puis homogénéisé de façon séquentielle, à 4°C, dans des volumes égaux des tampons suivants (trois fois dans chaque tampon : i) tampon TE : Tris-HCl 40 mM pH 7,5; éthylènediaminetétraacétate (EDTA) 10 mM; phénylméthylsulfonyl fluoride 0,25 mM et 2 µg/ml de chacun des inhibiteurs suivants: aprotinine, pepstatine A et leupeptine ; ii) tampon TENP40 : TE contenant un détergent, le Nonidet P-40 à 0,5% ; iii) tampons TEU : TE contenant différentes concentrations d'urée (de 2 à 8 M). Après chaque extraction, les homogénats ont été centrifugés pendant 15 minutes à 15 000 g et les surnageants prélevés. Le premier surnageant de chaque extraction a été gardé à -30°C jusqu'à utilisation. Finalement, le culot correspondant à la dernière extraction dans le tampon contenant de l'urée à 8M a été homogénéisé dans le tampon TUDTT: Tris-HCI 35 mM pH 6,8; urée 8 M; dithiothréitol 50 mM; glycérol 5%; phénylméthylsulfonyl fluoride 0,25 mM et 2 µg/ml de chacun des mêmes inhibiteurs, incubé 30 minutes à 95°C et centrifugé comme précédemment. Les concentrations protéiques ont été mesurées en utilisant le système de Pierce (Coomassie Plus protein assay, Pierce Chemical Co., Rockford, IL).

### Electrophorèse des protéines et immunodétection :

Les protéines ont été séparées par électrophorèse en gel de polyacrylamide en présence de SDS (PAGE-SDS) dans des gels de 7,5 ou 10% d'acrylamide, ou par électrophorèse bidimensionelle en présence d'urée, en utilisant le système de Pharmacia (PhastSystem™; Pharmacia LKB). Une isoélectrofocalisation (IEF) ou une électrophorèse interrompue avant l'équilibre de pH (NEpHGE: Non-Equilibrium pH Gel Electrophoresis) ont été réalisées en première dimension, puis une PAGE-SDS a été réalisée en deuxième dimension avec des gels de 12,5% de polyacrylamide. Pour les électrophorèses bidimensionelles, les protéines des extraits en tampon TENP40 ont été précipitées à l'éthanol, collectées par centrifugation et dissoutes dans 50 mM de Tris-HCl pH 7,4; 8 M d'urée et 0,5% de β-mercaptoéthanol. Les protéines marqueurs ainsi que les standards d'électrophorèse bidimensionelle (2-D SDS-PAGE standards™) de Bio-Rad ont été utilisés comme marqueurs de poids moléculaires ou comme références de points isoélectriques.
Après électrophorèse, les protéines ont été colorées au bleu de Coomassie ou à l'aide de nitrate d'argent (silver stain plus kit Bio-Rad Lab.), ou transférées sur membrane de nitrocellulose renforcée (Schleicher & Schuell, Dassel, Allemagne). Les membranes ont ensuite été colorées avec du rouge Ponceau ou du Protogold (British BioCell International, Cardiff, UK) et immunodétectées avec les anticorps monoclonaux, comme précédemment décrit. Les réactivités ont été révélées avec le kit ECL™ d'Amersham (ECL™ Western blotting kit, Amersham International, Aylesbury, UK), selon le protocole du fabriquant.

### Résultats :

Le polypeptide de 52-56 kDa a été détecté dans les extraits en tampons TE et TEU (à partir d'une concentration d'urée égale à 6 M), mais pas dans les extraits séquentiels en tampons TENP40 et TUDTT. De plus, si l'extrait en tampon TE était centrifugé pendant 30 minutes à 100 000 x g, Le polypeptide était totalement retrouvé dans le surnageant. L'anticorps monoclonal G36-19 a aussi reconnu plusieurs polypeptides de poids moléculaire apparent plus faible, qui n'étaient que partiellement extraits en présence d'urée (même à la concentration de 8 M), et étaient partiellement extraits en présence d'un agent réducteur. Que ce soit en tampon TE ou en présence d'urée, aucun polypeptide n'était immunodétectable dans le dernier des trois extraits réalisés. Ceci indique qu'à chacune des étapes, l'extraction était complète. Les expériences de contrôle réalisées en absence d'anticorps primaire ou avec l'anticorps MOPC-21 se sont toujours révélées négatives. Il est probable que les formes de polypeptides de faible poids moléculaire immunodétectées n'étaient pas des produits de dégradation générés durant les étapes d'extraction, puisque leur proportion ne variait pas d'une préparation à l'autre et puisque les extractions ont toujours été réalisées en présence d'inhibiteurs de protéases.

### La cornéodesmosine humaine est un composant des enveloppes cornifiées :

Afin de confirmer au niveau biochimique que la cornéodesmosine est liée de façon covalente aux enveloppes cornifiées, des fragments, générés par protéolyse d'enveloppes hautement purifiées à partir d'épiderme plantaire humain, ont été analysés avec l'anticorps monoclonal G36-19.

### Préparation et analyse des enveloppes cornifiées :

Des enveloppes cornifiées humaines ont été purifiées à partir de la couche cornée plantaire et d'épiderme mammaire, comme décrit précédemment. Brièvement, les échantillons ont été extraits par ébullitions répétées avec agitation vigoureuse dans une solution contenant 2% de SDS (p/v) et du dithiothréitol à la concentration de 25 mM, puis à 37°C pendant 72 heures dans une solution contenant de l'urée à la concentration de 8 M et du dithiothréitol à la concentration de 25 mM. Les enveloppes extraites en urée ont été sédimentées, puis suspendues dans une solution contenant 0,1% de SDS, de la glycine à la concentration de 192 mM et du Tris à la concentration de 125 mM. Enfin, elles ont été électrodialysées contre ce même tampon pendant 72 heures. Les enveloppes cornifiées purifiées ont été collectées par centrifugation, lavées à l'eau distillée et comptées. Elles ont été analysées morphologiquement, puis par immunofluorescence indirecte, comme décrit dans l'art antérieur. Une digestion à la protéase V8 et une immunodétection des produits de protéolyse ont été réalisées comme décrit dans l'art antérieur.

### Résultats :

Les enveloppes ont été incubées pendant des temps croissants avec la protéase V8, et les fragments produits ont été séparés par SDS-PAGE et immunodétectés. L'anticorps monoclonal G36-19 a fortement marqué un grand nombre de bandes de poids moléculaire apparent supérieur à 50 kDa, dont certaines étaient localisées à la limite supérieure du gel. Ceci indique que la cornéodesmosine est incorporée à l'intérieur de structures hétéropolymèriques de grande taille résultant de la protéolyse de l'enveloppe. Plusieurs bandes colorées par le Protogold n'étaient pas immunodétectées, ce qui confirme la spécificité de la réaction. Aucune bande n'était clairement immunodétectée avec l'anticorps monoclonal G36-19 en absence de protéolyse, ce qui prouve l'existence de liens covalents entre la cornéodesmosine et d'autres constituants des enveloppes cornifiées.

Aucun des fragments produits par digestion d'enveloppes cornifiées purifiées à partir d'épiderme mammaire n'a été immunodétecté par l'anticorps monoclonal G36-19. En accord avec ce résultat, ces enveloppes étaient beaucoup moins marquées, et seulement à leur périphérie, quand elles étaient analysées avec l'anticorps monoclonal G36-19 en immunofluorescence indirecte.

### La cornéodesmosine humaine de 52-56 kDa est une phosphoprotéine basique :

Pour déterminer le point isoélectrique de la cornéodesmosine, de l'épiderme humain a été extrait directement dans un tampon contenant un détergent (tampon TENP40), et les protéines extraites ont été séparées par une électrophorèse bidimensionelle (NEpHGE/SDS-PAGE) et immunodétectées avec l'anticorps monoclonal G36-19. La cornéodesmosine de 52-56 kDa a montré un point isoélectrique basique supérieur à 8. Ce résultat a été confirmé par immunodétection après une séparation IEF/SDS-PAGE. La protéine immunodétectée par l'anticorps monoclonal G36-19 n'était pas visible après coloration au bleu de Coomassie, même quand 100 µg de protéines de l'extrait en tampon TENP40 étaient chargés sur le gel. Ceci suggère donc que la cornéodesmosine de 52-56 kDa est une protéine quantitativement mineure, représentant moins de 0,1% des protéines extraites. De plus, la protéine immunoréactive migrait sous la forme de plusieurs taches juxtaposées, suggérant la présence de modifications post-traductionnelles. Pour analyser l'éventuelle phosphorylation de la cornéodesmosine humaine, celle-ci a été purifiée par affinité sur une matrice de sépharose (HiTrap-NHS, Pharmacia) activée par des groupements N-hydroxysuccinimide puis couplée avec l'anticorps monoclonal G36-19. Puis, elle a été analysée par immunotransfert avec des anticorps spécifiques de la phosphosérine ou de la phosphotyrosine. L'un des cinq anticorps monoclonaux anti-phosphosérine utilisés a immunodétecté la cornéodesmosine de façon spécifique. Au contraire, ni l'anticorps monoclonal PY20 ni un antisérum, dirigés contre la phosphotyrosine, n'ont reconnu la cornéodesmosine.

### La cornéodesmosine humaine de 52-56 kDa est une glycoprotéine :

### Affinodétection avec les lectines :

La cornéodesmosine de 52-56 kDa a été partiellement purifiée à partir d'un extrait en tampon TENP40 par chromatographie d'affinité. La cornéodesmosine fixée a été éluée avec 3% de SDS, déposée sur gel d'électrophorèse et transférée sur des membranes de nitrocellulose. Les membranes ont été incubées dans un tampon de blocage, puis avec des lectines biotinylées vendues par Pierce Chemical Co., diluées à 1 µg/ml: agglutinine de Pisum sativum (PSA), de germe de blé (WGA), de Ricinus communis (RCA), de Dolichos bifluorus (DBA) et de Arachis hypogaea (PNA). Après rinçage, les lectines ont été détectées avec de la streptavidine marquée avec de la peroxydase (diluée au 1/400 000), et le kit ECL™ d'Amersham, comme décrit plus haut.

### Expériences de déglycosylation :

L'extrait en tampon TENP40 (10 µg) a été maintenu à ébullition 3 minutes dans 20 µl d'un tampon phosphate de sodium à pH 7,2 contenant 1% (p/v) de SDS. Du Nonidet P40 et de l'EDTA ont été ajoutés pour atteindre une concentration finale de, respectivement, 1% et 20 mM. 2,4 unités de N-glycosidase F (EC 3.2.2.18, Boehringer Mannheim) ont été ajoutées et le mélange réactionnel a été incubé à 37°C pendant 6 heures. Les protéines (34 µg) d'un extrait en tampon TENP40 ont aussi été incubées avec 5 mU d'endo-α-N-acétylgalactosaminidase (EC 3.2.1.97) à 37 °C pendant 6 heures, en présence ou non de N-glycosidase F ou de neuraminidase (EC 3.2.1.18), dans les conditions décrites par le fabricant (Oxford GlycoSystems Ltd, Abingdon, UK). Les réactions ont été arrêtées par 2 minutes d'ébullition dans du tampon échantillon. La déglycosylation de la fétuine, utilisée comme contrôle positif, a été testée par PAGE-SDS et avec les lectines biotinylées. Les échantillons traités et non traités on été séparés par électrophorèse et analysés par immunodétection et affinodétection, comme décrit plus haut.

### Chromatographie sur Concanavaline A-Sépharose :

Un extrait en tampon TENP40 a été directement injecté, avec un débit de 0,5 ml/minute, sur une colonne de Concanavaline A sépharose 4B (ConA Sepharose, Sigma) qui avait été équilibrée au préalable avec du tampon de lavage: Tris-HCl 20 mM pH 7,4 contenant 0,2 M de NaCl. Après lavage, avec un débit de 1 ml/minute, avec 15 ml de ce tampon, les protéines adsorbées ont été éluées avec un débit de 0,5 ml/minute avec du méthyl-α-D mannopyranoside (Sigma) dilué à 0,5 M dans le tampon de lavage. Les protéines ont ensuite été séparées par PAGE-SDS et analysées comme décrit plus haut.

### Résultats :

Les protéines d'un extrait en tampon TENP40, contenant la cornéodesmosine de 52-56 kD, ont été traitées par diverses glycosidases et analysées en immunotransfert avec deux anticorps monoclonaux dirigés contre le polypeptide. La coloration des protéines totales de l'extrait n'a pas montré de dégradation apparente durant l'incubation. Le traitement par la N-glycosidase F a induit une diminution d'environ 5 kDa du poids moléculaire apparent de la cornéodesmosine. Ce résultat suggère très fortement une N-glycosylation. Au contraire, les traitements avec l'endo-a-N-acétylgalactosaminidase et/ou la neuraminidase n'ont pas modifié la migration du la cornéodesmosine. Pour confirmer ce résultat, la cornéodesmosine humaine a été purifiée par affinité et analysée à l'aide de lectines biotinylées. L'agglutinine de germe de blé (WGA) a fortement lié la protéine purifiée, à l'inverse des autres lectines testées qui ne l'ont pas ou très faiblement reconnue. De plus, la cornéodesmosine s'est liée à de la concanavaline A couplée à une matrice de sépharose. Elle a pu être éluée avec le méthyl α-D-mannopyranoside, sucre spécifique de cette lectine, à la concentration de 0,5 M, ce qui prouve la spécificité de la liaison.

### Purification de la cornéodesmosine :

Après clivage dermo-épidermique, l'épiderme a été homogénéisé dans un tampon TEA: Tris-HCl 40 mM pH 7,5; EDTA 10 mM; aprotinine 10 µg/ml et 4-(2-aminoéthyl)-benzènesulfonyl fluoride (Interchim, Paris, France) utilisé à 0,8 mM. L'homogénat a été centrifugé pendant 15 minutes à 15000 g. Le surnageant a été clarifié par filtration sur des filtres dont le diamètre des pores est de 0,45 µm (Puradisc ™ 25 AS; Whatman, Clifton, NJ), et injecté avec un débit de 0,3 ml/minute sur une colonne échangeuse d'anion (Hi Trap Q, Pharmacia LKB) qui avait été équilibrée dans le tampon de lavage: Tris-HCl 20 mM pH 7,5. Les protéines non retenues (soit environ 5% des protéines totales de l'extrait) ont été directement injectées au même débit sur une colonne d'affinité préparée comme suit: environ 2 mg de l'anticorps monoclonal F28-27 ont été couplés à 1 ml de matrice de Sépharose 4B activée par des groupements N-hydroxysuccinimide (HiTrap-NHS), comme cela est préconisé par le fabricant Pharmacia LKB. La colonne a été lavée de façon exhaustive avec un débit de 1 ml/minute avec le tampon de lavage en présence puis en absence de 1 M de NaCl. Les protéines immunoadsorbées ont été éluées avec un débit de 0,3 ml/minute avec 0,2 M de glycine à pH 2,5; le pH des fractions éluées a été immédiatement neutralisé avec du Tris base à la concentration de 2 M. Les protéines des différentes fractions ont été analysées en immunotransfert avec l'anticorps monoclonal G36-19, ou avec l'anticorps monoclonal de contrôle, MOPC-21. Les fractions contenant les protéines ainsi éluées ont été mélangées, puis lyophilisées. Les protéines du lyophilisat ont été analysées comme décrit plus haut, par électrophorèse mono- ou bidimensionelle. La cornéodesmosine a été ensuite découpée du gel afin d'être séquencée.

### Résultats :

La cornéodesmosine de 52-56 kDa a été purifiée par une chromatographie d'échange d'anions suivie d'une chromatographie d'affinité. La totalité de la cornéodesmosine extraite s'est liée de façon spécifique sur la colonne d'affinité, dont elle a pu être éluée en présence de glycine. La coloration au Protogold des protéines transférées montre le haut degré de purification obtenu, puisque la cornéodesmosine est largement majoritaire dans ces fractions. Des résultats similaires ont été obtenus, lorsque la chromatographie d'affinité était réalisée sur une matrice couplée avec l'anticorps monoclonal F28-27 ou avec l'anticorps monoclonal G36-19. Une analyse en gel bidimensionel a confirmé le point isoélectrique basique de la cornéodesmosine purifiée et a montré qu'elle est la seule protéine éluée présentant un poids moléculaire apparent de 52-56 kDa. La cornéodesmosine ainsi purifiée a été séparée par électrophorèse et la bande du gel contenant cette protéine a été découpée, puis la protéine a été séquencée.

### Instabilité du polypeptide purifié :

Au cours de ses nombreux essais pour purifier la cornéodesmosine, la demanderesse s'est heurtée à deux problèmes majeurs : (1) la faible proportion de cette protéine parmi les protéines épidermiques et (2) son instabilité, due vraisemblablement à sa grande sensibilité à l'action des protéases.
Différents tests ont été mis en oeuvre pour tenter de stabiliser la cornéodesmosine : adjonction de zinc (inhibiteur de l'enzyme chymotrypsique de la couche cornée), d'agents réducteurs (β-mercaptoéthanol ou dithiothréitol), d'agents dénaturants (SDS), de glycérol, d'inhibiteurs spécifiques des protéases à sérine (PMSF, aprotinine) ou d'un cocktail d'inhibiteurs de diverses protéases (aprotinine, leupeptine, pepstatine, benzamidine, phénanthroline, PMSF) ou suppression des étapes de congélation / décongélation. Finalement, l'absence des étapes de congélation/décongélation, la présence de 2% de SDS ou la présence du cocktail d'inhibiteurs, et de préférence une combinaison de ces conditions, se sont révélées les plus efficaces pour stabiliser la cornéodesmosine.

### Séquençage du polypeptide purifié :

Pour le séquençage, les bandes qui correspondent à la cornéodesmosine ou à des fragments de cornéodesmosine (52-56 kDa, 45 kDa), mises en évidence sur les gels d'electrophorèse ont été découpées. Les protéines ont alors été directement digérées dans le gel par de l'endopeptidase lys-C (EC 3.4.99.30) et les peptides générés ont été purifiés par HPLC en utilisant une colonne DEAE-C18. Les peptides ainsi sélectionnés ont été séquencés par la méthode des cycles de dégradation d'Edman sur un appareil Procise Sequencer de la société Applied Biosystems, selon lés instructions du fournisseur.

### Résultats :

Aucun séquençage à partir de l'extrémité NH2-terminale n'a été possible, suggérant une protection de la protéine à cette extrémité.
Les séquençages internes ont permis d'établir la séquence de deux fragments à savoir :

A : Lys Ser Tyr Gly Gly Tyr Glu Val Val Gly Gly Ser Ser Asp Ser Gly
B : Lys Ile Tyr Pro Val Gly Tyr Phe Thr Lys

Ce résultat confirme celui obtenu par les techniques de biologie moléculaire ayant permis la déduction de la séquence en acides aminés du polypeptide de l'invention à partir de la séquence nucléotidique.

### Construction d'une banque d'expression d'épiderme humain :

Le clonage de l'ADNc de la cornéodesmosine humaine ne pouvait pas être réalisé à partir des banques d'expression préexistantes. En effet, celles-ci ont été produites soit à partir d'épiderme de souris, soit à partir de kératinocytes humains cultivés en monocouche, conditions qui ne permettent pas l'expression des gènes caractéristiques de la différenciation terminale épidermique. La demanderesse a donc construit une banque d'expression épidermique humaine.

### Extraction d'acides ribonucléiques (ARN) poly(A)⁺ d'épiderme humain :

Elle a été réalisée à partir de prélèvements de peau mammaire, déchets chirurgicaux obtenus après chirurgie plastique. Le tissu adipeux sous-cutané a été disséqué, puis des lambeaux de peau ont été découpés à l'aide d'un dermatome et incubés 2 heures à 37°C, dans une solution de trypsine (solution A, Gibco BRL), face épidermique vers le haut. Le clivage dermo-épidermique a été alors réalisé avec des pinces. Les feuillets épidermiques obtenus ont été rincés dans un tampon phosphate (PBS, pH 7,4). L'extraction des ARN poly(A)⁺ a été réalisée selon le protocole proposé dans le coffret "mRNA purification kit" commercialisé par Dynal (Oslo), après homogénéisation des feuillets à l'aide d'un "Turax", directement dans le tampon fourni (Lysis/Binding buffer). Le principe de ce coffret utilise l'affinité des ARN messagers (ARNm) comportant une extrémité poly(A) pour des billes magnétiques recouvertes d'oligo(dT)₂₅. Après 5 minutes d'incubation à température ambiante dans le lysat, les billes ont été isolées avec un aimant et soumises à 3 lavages. Les ARN poly(A)⁺ élués par incubation des billes à 65°C pendant 2 minutes dans une solution 2 mM EDTA, pH 8, ont été dosés en utilisant le coffret colorimétrique "DNA DipStick" commercialisé par Invitrogen (San Diego, CA).

### Construction de la banque :

La banque a été construite avec le coffret "ZAP Express cDNA Gigapack II Gold cloning kit" commercialisé par Stratagene (La Jolla, CA), en suivant le protocole proposé par le fournisseur. Les ADN complémentaires (ADNc) ont été synthétisés à partir de 2 µg d'ARN poly(A)⁺, avec la transcriptase inverse du virus de la leucémie murine de Moloney (MMLV-RT), en utilisant des amorces oligo(dT)18 comprenant un site de restriction pour l'enzyme Xhol et en présence de 5-méthyl cytidine triphosphate. La synthèse du second brin a été réalisée par l'ADN polymérase I de E. coli, et les extrémités des ADNc ont été rendues franches avec la pfu polymérase recombinante. Après addition d'adaptateurs EcoR I (ADN ligase de T4), et phosphorylation des extrémités 5' (polynucléotide kinase de T4), les ADNc ont été soumis à une digestion par l'enzyme Xho I. La sélection des ADNc de taille supérieure à 500 paires de bases a été réalisée avec une colonne de Sephacryl S-500. La ligation des ADNc avec les deux bras du phage ZAP Express a été réalisée avec la ligase du phage T4. L'encapsulation du phage recombinant a été réalisée avec les extraits "Gigapack II Gold Packaging Extract" fournis par Stratagene. La souche XL-1 blue MRF' de E. coli a été utilisée pour la titration et l'étalement de la banque.

### Résultats :

Cette banque a été réalisée à partir de 2 µg d'ARNm, extraits d'un fragment d'épiderme humain dans les heures suivant son prélèvement, par clonage unidirectionnel dans le phage ZAP Express. Elle est constituée d'environ 2x10⁵ clones indépendants.

### Clonage de l'acide désoxyribonucléique complémentaire (ADNc) codant pour la cornéodesmosine :

### Criblage immunologique :

Le criblage immunologique, réalisé sans étape d'amplification préalable, a été fait sur des membranes de nitrocellulose (Schleicher & Schuell, Dassel, Allemagne), incubées dans une solution d'isopropyl-1-thio-β-D-galactopyranoside (IPTG, Stratagene) à 10 mM pendant 10 minutes, séchées, et incubées 4h à 37°C sur la banque étalée. La procédure de criblage immunologique a été réalisée comme décrit dans l'art antérieur avec un cocktail des trois anticorps monoclonaux G36-19, F28-27 et B17-21, utilisés aux concentrations respectives de 0,2, 0,2 et 2 µg/ml. Les clones positifs ont été isolés par les techniques classiques et testés avec chacun des trois anticorps monoclonaux isolément.

### Séquençage :

Les phages ZAP Express correspondant aux clones purifiés ont été soumis à une excision *in vivo,* avec l'aide d'un phage ExAssist, comme indiqué par Stratagene. Les plasmides obtenus ont été amplifiés avec le coffret Qiagen (Hilden, Allemagne), puis séquencés aux extrémités des inserts, avec des amorces T3 et M13 (Stratagene), en utilisant un coffret Perkin Elmer (ABI PRISM Dye Terminator cycle sequencing kit, Perkin Elmer, Foster city, CA). La comparaison des séquences obtenues avec les bases de données internationales a été réalisée avec le programme Blast.

### Résultats :

La banque n'ayant pas été amplifiée préalablement, le criblage a permis d'isoler 5 clones indépendants. Les fragments d'ADNc humain contenus dans les clones ont été séquencés partiellement à partir des extrémités, ce qui a permis de montrer qu'ils étaient tous chevauchants et codaient donc tous pour des fragments de la même protéine. Les 5 clones ont aussi été testés avec chacun des trois anticorps monoclonaux séparément. Trois clones (1.1, 4.4 et 5.1) se sont révélés réactifs avec les trois anticorps monoclonaux, le clone 5.5 avec deux anticorps monoclonaux (F28-27 et G36-19) et enfin le clone 1.2 n'a été reconnu que par l'anticorps monoclonal F28-27. Ces résultats démontrent de manière formelle que les trois anticorps monoclonaux reconnaissent la même protéine, confirmant ainsi ce qui avait été fortement suggéré par les études biochimiques antérieures. Ces résultats permettent également d'ordonner sur la molécule les épitopes reconnus par chacun des anticorps monoclonaux. En effet, les clones ayant été construits par transcription inverse à partir de l'extrémité 3' des ARNm, qui correspond à l'extrémité COOH de la protéine, il est possible de conclure que l'épitope reconnu par F28-27 est le plus proche de cette extrémité, suivi de l'épitope de G36-19 et enfin de celui de B17-21. L'analyse par restriction enzymatique de la taille de ces différents fragments d'ADNc, a confirmé que les 3 clones reconnus par les 3 anticorps monoclonaux présentent les plus longues séquences (de 2 à 1,5 kb), alors que celui que reconnaît seulement F28-27 est le plus court (1 kb), le dernier, reconnu par 2 anticorps monoclonaux, ayant une taille intermédiaire de 1,3 kb. Ainsi, a été cloné tout ou partie de l'ADNc codant pour la cornéodesmosine humaine, protéine caractérisée par les 3 anticorps monoclonaux B17-21, G36-19 et F28-27 qui reconnaissent des épitopes différents, ordonnés sur la protéine dans l'ordre indiqué, de l'extrémité NH2 vers l'extrémité COOH.

Les séquences partielles de chaque clone, comparées aux séquences incluses dans les banques de données internationales, ont révélé une identité de 99% avec la séquence du gène S, inscrit dans la banque de données GenBank sous le numéro L20815. Cette analyse a montré également que parmi les 5 clones obtenus, 4 correspondent à la forme courte de l'ARNm, un seul, le clone 5.1, correspondant à la forme longue. Ces formes sont issues d'un choix alternatif du site de polyadénylation au niveau de la partie 3' non codante du transcrit primaire. Cette analyse a permis de préciser la localisation des épitopes reconnus par nos 3 anticorps monoclonaux : l'épitope reconnu par B17-21 est localisé dans la partie de la protéine correspondant aux nucléotides 594 à 762, G36-19 et F28-27 étant respectivement localisés dans la zone 762-1044 et 1044-codon stop. Enfin, le clone 1.1, qui comporte la séquence codante la plus longue, commence au nucléotide 348.

### Clonage de l'ADNc complet codant pour la cornéodesmosine :

Les analyses précédentes ont montré que les clones obtenus ne couvraient pas la partie de l'ADNc allant du nucléotide 1 au nucléotide 347. La demanderesse a donc dû cloner cette partie manquante.
80 ng d'ARN poly(A)⁺ extrait d'épiderme humain comme précédemment décrit, ont été soumis à une transcription inverse à partir d'amorces aléatoires, en utilisant le coffret "SuperScript" commercialisé par Gibco BRL. L'amplification de l'extrémité 5' de l'ADNc de la cornéodesmosine a été réalisée à partir de 1/20 de la réaction de synthèse du premier brin, avec en amont un oligonucléotide correspondant à la séquence 2-20 (GenBank L20815) et comprenant aussi un adaptateur Spe I, et, en aval un oligonucléotide complémentaire de la séquence 1017-1035. Les conditions de la réaction de polymerisation en chaîne (PCR : polymerase chain reaction) étaient les suivantes : 3 minutes à 94°C suivies de 35 cycles avec 80 sec à 94°C, 80 sec à 57°C et 2 minutes à 72°C. Le fragment unique obtenu, purifié après électrophorèse en gel d'agarose TBE, digéré par Spe I et EcoN I (nucléotides 2 à 1003), a été cloné dans le plasmide pBK-CMV-1.1 (isolé par criblage de la banque) digéré par les mêmes enzymes, ce qui a permis d'aboutir au plasmide pS11. L'ADNc complet contenu dans le plasmide pS11 a été séquencé au moins 3 fois jusqu'au nucléotide 1700. La comparaison avec la séquence antérieurement publiée a révélé quatre différences ponctuelles qui ont été analysées au niveau génomique.

### Analyse génomique de la cornéodesmosine :

Quatre différences ponctuelles ont été constatées entre la séquence de l'ADNc complet isolé par la demanderesse et celle du gène S publiée dans l'art antérieur (Zhou Y. et Chaplin D.D., P.N.A.S. usa, Vol. 90, pp. 9470-9474, Octobre 1993). Ces quatre différences ponctuelles ont été analysées par PCR, au niveau génomique à partir de dix échantillons témoins d'ADN extrait de sang humain selon les méthodes classiques.
L'analyse de l'insertion éventuelle d'une guanine en position 1514 a été réalisée après amplification par PCR de la région génomique comprise entre les nucléotides 1446 et 1786 (GenBank, L20815), réalisée dans les conditions classiques. Le fragment d'ADN obtenu pour chaque échantillon a été soumis à une digestion par Bsi MI d'une part et Nci I d'autre part. Une électrophorèse sur gel NuSieve GTG 3% (FMC, Rockland, ME) a été réalisée en tampon TBE. L'étude de la séquence en position 66 a été réalisée selon la méthode ASA (allele specific amplification). Deux réactions de PCR ont été réalisées en parallèle avec en aval, un oligonucléotide complémentaire de la séquence 572-91 et, en amont, un oligonucléotide correspondant à la séquence 52-65 et se terminant en position 66 soit par A soit par T. Les réactions de PCR ont été réalisées avec 0,5 µg d'ADN génomique, 2,5 pmol de chaque oligonucléotide, et 0,5 U de Taq polymérase (Appligene). Les conditions d'amplification sont 94°C, 2 minutes puis 30 cycles avec 50 secondes à 94°C, 1 minute à 56°C et 2,5 minutes à 72°C.
L'analyse des bases en position 1118 et 1236 a été réalisée après amplification avec un couple d'oligonucléotides unique : oligonucléotide sens 908-27, antisens 1573-93. Les conditions de PCR sont les mêmes que précédemment, avec un temps de polymérisation limité à 1 minute. Les fragments ont été soumis à une digestion par Bsm I ou Hin6 I pour l'étude respective des positions 1118 et 1236 puis séparés par électrophorèse sur gel NuSieve 3%.

### Résultats :

L'insertion d'une guanine en position 1514 entraîne un décalage du cadre de lecture et déplace le codon stop de la position 1523 à la position 1603. La protéine produite par cette séquence diffère au niveau des deux derniers acides aminés (I et S) qui sont remplacés par la séquence : 
Asp Ile leu Ala Gln Val lys Pro Leu Gly Pro Gln Leu Ala Asp Pro Glu Val Phe
Leu Pro Gln Gly Glu Leu Leu Asp Ser Pro
 ce qui correspond à un ajout de 27 acides aminés par rapport à la séquence publiée. Les 5 ADNc précédemment isolés présentant tous cette insertion, l'étude a été élargie à d'autres individus. Dix échantillons témoins d'ADN genomique humain, provenant de dix individus, ont été amplifiés dans cette région et analysés par restriction enzymatique. En effet la séquence publiée correspond à la présence d'un site de restriction Bsi MI en position 1510 qui disparaît dans la séquence comprenant une guanine supplémentaire, alors qu'un site Nci I apparaît en 1512. Les résultats sont les mêmes pour les 10 individus, à savoir que seul le site Nci I est présent. Ceci signifie que chez ces dix individus, la cornéodesmosine est produite sous une forme plus longue que la forme publiée du gène S.
Trois autres différences ponctuelles, de type substitutions, ont été retrouvées entre les clones et la séquence publiée. L'analyse des dix échantillons génomiques témoins par PCR et restriction enzymatique ou par ASA, a montré qu'il s'agit d'un polymorphisme du gène. La demanderesse a mis en évidence en position 66 une mutation conservative T/A (Leu-Met), en position 1118, un polymorphisme A/G, correspondant à une mutation silencieuse Ala-Ala et, enfin, en 1236 un polymorphisme T/G correspondant à une mutation non conservative Ser-Ala.

### Production de cornéodesmosine recombinante :

L'ADNc complet codant pour la cornéodesmosine a été isolé, sous la forme d'un fragment Ecl136 I/Not I de 2106 paires de bases à partir du plasmide pS11. Le sous-clonage a été réalisé dans le vecteur pCDNA amp (InVitrogen) préalablement digéré par EcoR V et Not I, aboutissant au plasmide p14-9. La traduction in vitro de la cornéodesmosine, a été effectuée avec le coffret "TNT T7 Quick coupled transcription/translation system" commercialisé par Promega, à partir du plasmide p14-9. Ce coffret associe en une étape, la transcription par l'ARN polymérase du phage T7, avec la traduction par un lysat de réticulocytes de lapin. Ces réactions ont été réalisées avec 0,6 µg de plasmide dans un volume de 25 µl, selon les instructions du fournisseur. La cornéodesmosine a été exprimée dans des cellules COS-7 après transfection avec le plasmide p14-9. Les cellules ont été transfectées selon le protocole classique au DEAE-dextran, complété par l'addition de chloroquine et un choc au DMSO, en utilisant 1 µg de plasmide/3.10⁵ cellules. Après 48 h d'expression, les cellules sont lavées 2 fois au PBS pH 7,4 et lysées dans un tampon Tris-HCl 40 mM pH 7,5, EDTA 10 mM, NP40 0,5%, contenant un cocktail d'inhibiteurs de protéases (Pharmingen, Inc). Après séparation en SDS-PAGE, et électrotransfert, les échantillons sont immunodétectés, comme précédemment décrit.

### Résultats :

La nouvelle séquence complète de la protéine prédit un poids moléculaire de 51,45 kDa, en absence de toute modification post-traductionnelle. La cornéodesmosine synthétisée *in vitro,* en absence de membranes microsomales, migre avec un poids moléculaire apparent d'environ 60 kDa. Ceci montre que cette protéine a une migration aberrante en gel SDS-PAGE. La co-migration avec la forme épidermique majoritaire extraite en tampon de faible force ionique, démontre que cette dernière correspond à une forme assez largement tronquée de la protéine, ceci d'autant plus que la demanderesse a montré que cette forme épidermique est glycosylée.
La transfection de cellules COS-7 avec le plasmide 14-9 fait apparaître une protéine immunoréactive qui migre également aux environs de 60 kDa. Les mêmes résultats ont été obtenus avec la lignée de neuroépithéliome humain CHP 126, transfectée par électroporation. L'ensemble de ces résultats suggère fortement que dans l'épiderme, la cornéodesmosine subit, de manière précoce au cours de sa maturation, une protéolyse spécifique.

## Revendications

1. Composition cosmétique ou pharmaceutique comprenant, dans un milieu physiologiquement acceptable, au moins un polypeptide naturel ou synthétique purifié, ledit polypeptide étant **caractérisé par le fait qu'**il a la séquence d'acides aminés SEQ ID NO : 1 suivante :

Met Gly Ser Ser Arg Ala Pro Trp Met Gly Arg Val Gly Gly His Gly
1               5                   10                  15
Met Met Ala Leu Leu Leu Ala Gly Leu Leu Leu Pro Gly Thr Leu Ala
            20                  25                  30
Lys Ser Ile Gly Thr Phe Ser Asp Pro Cys Lys Asp Pro Thr Arg Ile
        35                  40                  45
Thr Ser Pro Asn Asp Pro Cys Leu Thr Gly Lys Gly Asp Ser Ser Gly
    50                  55                  60
Phe Ser Ser Tyr Ser Gly Ser Ser Ser Ser Gly Ser Ser Ile Ser Ser
65                  70                  75                  80
Ala Arg Ser Ser Gly Gly Gly Ser Ser Gly Ser Ser Ser Gly Ser Ser
                85                  90                  95
Ile Ala Gln Gly Gly Ser Ala Gly Ser Phe Lys Pro Gly Thr Gly Tyr
            100                 105                 110
Ser Gln Val Ser Tyr Ser Ser Gly Ser Gly Ser Ser Leu Gln Gly Ala
        115                 120                 125
Ser Gly Ser Ser Gln Leu Gly Ser Ser Ser Ser His Ser Gly Ser Ser
    130                 135                 140
Gly Ser His Ser Gly Ser Ser Ser Ser His Ser Ser Ser Ser Ser Ser
145                 150                 155                 160
Phe Gln Phe Ser Ser Ser Ser Phe Gln Val Gly Asn Gly Ser Ala Leu
                165                 170                 175
Pro Thr Asn Asp Asn Ser Tyr Arg Gly Ile Leu Asn Pro Ser Gln Pro
            180                 185                 190
Gly Gln Ser Ser Ser Ser Ser Gln Thr Ser Gly Val Ser Ser Ser Gly
        195                 200                 205
Gln Ser Val Ser Ser Asn Gln Arg Pro Cys Ser Ser Asp Ile Pro Asp
    210                 215                 220
Ser Pro Cys Ser Gly Gly Pro Ile Val Ser His Ser Gly Pro Tyr Ile
225                 230                 235                 240
Pro Ser Ser His Ser Val Ser Gly Gly Gln Arg Pro Val Val Val Val
                245                 250                 255

Val Asp Gln His Gly Ser Gly Ala Pro Gly Val Val Gln Gly Pro Pro
            260                 265                 270
Cys Ser Asn Gly Gly Leu Pro Gly Lys Pro Cys Pro Pro Ile Thr Ser
        275                 280                 285
Val Asp Lys Ser Tyr Gly Gly Tyr Glu Val Val Gly Gly Ser Ser Asp
    290                 295                 300
Ser Tyr Leu Val Pro Gly Met Thr Tyr Ser Lys Gly Lys Ile Tyr Pro
305                 310                 315                 320
Val Gly Tyr Phe Thr Lys Glu Asn Pro Val Lys Gly Ser Pro Gly Val
                325                 330                 335
Pro Ser Phe Ala Ala Gly Pro Pro Ile Ser Glu Gly Lys Tyr Phe Ser
            340                 345                 350
Ser Asn Pro Ile Ile Pro Ser Gln Ser Ala Ala Ser Ser Ala Ile Ala
        355                 360                 365
Phe Gln Pro Val Gly Thr Gly Gly Val Gln Leu Cys Gly Gly Gly Ser
    370                 375                 380
Thr Gly Ser Lys Gly Pro Cys Ser Pro Ser Ser Ser Arg Val Pro Ser
385                 390                 395                 400
Ser Ser Ser Ile Ser Ser Ser Ala Gly Ser Pro Tyr His Pro Cys Gly
                405                 410                 415
Ser Ala Ser Gln Ser Pro Cys Ser Pro Pro Gly Thr Gly Ser Phe Ser
            420                 425                 430
Ser Ser Ser Ser Ser Gln Ser Ser Gly Lys Ile Ile Leu Gln Pro Cys
        435                 440                 445
Gly Ser Lys Ser Ser Ser Ser Gly His Pro Cys Met Ser Val Ser Ser
    450                 455                 460
Leu Thr Leu Thr Gly Gly Pro Asp Gly Ser Pro His Pro Asp Pro Ser
465                 470                 475                 480
Ala Gly Ala Lys Pro Cys Gly Ser Ser Ser Ala Gly Lys Ile Pro Cys
                485                 490                 495
Arg Ser Ile Arg Asp Ile Leu Ala Gln Val Lys Pro Leu Gly Pro Gln
            500                 505                 510
Leu Ala Asp Pro Glu Val Phe Leu Pro Gln Gly Glu Leu Leu Asp Ser
        515                 520                 525
Pro

2. Composition selon la revendication 1, **caractérisée par le fait que** le polypeptide est purifié à partir de mammifères.

3. Composition selon l'une quelconque des revendications précédentes, **caractérisée par le fait que** le polypeptide est purifié à partir de peau de mammifères.

4. Composition selon l'une quelconque des revendications précédentes, **caractérisée par le fait que** le polypeptide est purifié à partir de peau humaine.

5. Composition selon l'une quelconque des revendications précédentes, **caractérisée par le fait que** le polypeptide est purifié à partir d'épiderme humain.

6. Composition selon l'une quelconque des revendications précédentes, **caractérisée par le fait que** le polypeptide intervient dans la cohésion intercornéocytaire.

7. Composition selon l'une quelconque des revendications précédentes, **caractérisée par le fait que** le polypeptide est en partie constituée par la séquence du polypeptide tel que décrit dans la revendication 1.

8. Composition selon l'une quelconque des revendications précédentes, **caractérisée par le fait que** le polypeptide a un poids moléculaire apparent, tel qu'il peut être déterminé par électrophorèse en gel de polyacrylamide SDS (PAGE-SDS), compris entre 50 et 60 kilodaltons, préférentiellement entre 52 et 56 kilodaltons.

9. Composition selon l'une quelconque des revendications précédentes, **caractérisée par le fait que** le polypeptide est basique.

10. Composition selon l'une quelconque des revendications précédentes, **caractérisée par le fait que** le polypeptide est glycosylé.

11. Composition selon l'une quelconque des revendications précédentes, **caractérisée par le fait que** le polypeptide est phosphorylé.

12. Composition selon l'une quelconque des revendications précédentes, **caractérisée par le fait que** le polypeptide est glycosylé, basique, phosphorylé et que le polypeptide a un poids moléculaire apparent, tel qu'il peut être déterminé par électrophorèse en gel de polyacrylamide SDS (PAGE-SDS), compris entre 50 et 60 kilodaltons, préférentiellement entre 52 et 56 kilodaltons.

13. Composition selon l'une quelconque des revendications 1 à 12, destinée à traiter l'amincissement de l'épiderme et en particulier de la couche cornée et/ou à traiter une fragilité excessive du revêtement cutané et/ou à renforcer la cohésion intercornéocytaire et/ou induire l'épaississement de la couche cornée.

14. Composition selon l'une quelconque des revendications 1 à 12, destinée à traiter les troubles trophiques cutanés ou ceux consécutifs à des troubles de la cicatrisation.

15. Utilisation d'au moins un polypeptide de séquence SEQ ID NO : 1 pour la préparation d'une composition cosmétique selon l'une quelconque des revendications 1 à 12, ladite composition étant destinée au traitement cosmétique de l'amincissement de l'épiderme, et en particulier de la couche cornée, et/ou pour traiter une fragilité excessive du revêtement cutané et/ou pour renforcer la cohésion intercornéocytaire et/ou induire l'épaississement de la couche cornée, ladite composition convenant à une application sur la peau du sujet à traiter.

16. Utilisation d'au moins un polypeptide de séquence SEQ ID NO : 1 pour la préparation d'une composition cosmétique selon l'une quelconque des revendications 1 à 12, ladite composition étant destinée au traitement des troubles trophiques cutanés ou ceux consécutifs à des troubles de la cicatrisation, et convenant à une application sur la peau du sujet à traiter.

17. Composition cosmétique ou pharmaceutique comprenant, dans un milieu physiologiquement acceptable, au moins la séquence nucléotidique codante SEQ ID N°2 suivante :

1 ATG GGC TCG TCT CGG GCA CCC TGG ATG GGG CGT GTG GGT GGG CAC
46 GGG ATG ATG GCA CTG CTG CTG GCT GGT CTC CTC CTG CCA GGG ACC
91 TTG GCT AAG AGC ATT GGC ACC TTC TCA GAC CCC TGT AAG GAC CCC
136 ACG CGT ATC ACC TCC CCT AAC GAC CCC TGC CTC ACT GGG AAG GGT
181 GAC TCC AGC GGC TTC AGT AGC TAC AGT GGC TCC AGC AGT TCT GGC
226 AGC TCC ATT TCC AGT GCC AGA AGC TCT GGT GGT GGC TCC AGT GGT
271 AGC TCC AGC GGA TCC AGC ATT GCC CAG GGT GGT TCT GCA GGA TCT
316 TTT AAG CCA GGA ACG GGG TAT TCC CAG GTC AGC TAC TCC TCC GGA
361 TCT GGC TCT AGT CTA CAA GGT GCA TCC GGT TCC TCC CAG CTG GGG
406 AGC AGC AGC TCT CAC TCG GGA AGC AGC GGC TCT CAC TCG GGA AGC
451 AGC AGC TCT CAT TCG AGC AGC AGC AGC AGC TTT CAG TTC AGC AGC
496 AGC AGC TTC CAA GTA GGG AAT GGC TCT GCT CTG CCA ACC AAT GAC
541 AAC TCT TAC CGC GGA ATA CTA AAC CCT TCC CAG CCT GGA CAA AGC
586 TCT TCC TCT TCC CAA ACC TCT GGG GTA TCC AGC AGT GGC CAA AGC
631 GTC AGC TCC AAC CAG CGT CCC TGT AGT TCG GAC ATC CCC GAC TCT
676 CCC TGC AGT GGA GGG CCC ATC GTC TCG CAC TCT GGC CCC TAC ATC
721 CCC AGC TCC CAC TCT GTG TCA GGG GGT CAG AGG CCT GTG GTG GTG
766 GTG GTG GAC CAG CAC GGT TCT GGT GCC CCT GGA GTG GTT CAA GGT
811 CCC CCC TGT AGC AAT GGT GGC CTT CCA GGC AAG CCC TGT CCC CCA
856 ATC ACC TCT GTA GAC AAA TCC TAT GGT GGC TAC GAG GTG GTG GGT
901 GGC TCC TCT GAC AGT TAT CTG GTT CCA GGC ATG ACC TAC AGT AAG
946 GGT AAA ATC TAT CCT GTG GGC TAC TTC ACC AAA GAG AAC CCT GTG

991 AAA GGC TCT CCA GGG GTC CCT TCC TTT GCA GCT GGG CCC CCC ATC
1036 TCT GAG GGC AAA TAC TTC TCC AGC AAC CCC ATC ATC CCC AGC CAG
1081 TCG GCA GCT TCC TCG GCC ATT GCG TTC CAG CCA GTG GGG ACT GGT
1126 GGG GTC CAG CTC TGT GGA GGC GGC TCC ACG GGC TCC AAG GGA CCC
1171 TGC TCT CCC TCC AGT TCT CGA GTC CCC AGC AGT TCT AGC ATT TCC
1216 AGC AGC GCC GGT TCA CCC TAC CAT CCC TGC GGC AGT GCT TCC CAG
1261 AGC CCC TGC TCC CCA CCA GGC ACC GGC TCC TTC AGC AGC AGC TCC
1306 AGT TCC CAA TCG AGT GGC AAA ATC ATC CTT CAG CCT TGT GGC AGC
1351 AAG TCC AGC TCT TCT GGT CAC CCT TGC ATG TCT GTC TCC TCC TTG
1396 ACA CTG ACT GGG GGC CCC GAT GGC TCT CCC CAT CCT GAT CCC TCC
1441 GCT GGT GCC AAG CCC TGT GGC TCC AGC AGT GCT GGA AAG ATC CCC
1486 TGC CGC TCC ATC CGG GAT ATC CTA GCC CAA GTG AAG CCT CTG GGG
1531 CCC CAG CTA GCT GAC CCT GAA GTT TTC CTA CCC CAA GGA GAG TTA
1576 CTC GAC AGT CCA TAA

## Claims

1. Cosmetic or pharmaceutical composition comprising, in a physiologically acceptable medium, at least one purified natural or synthetic polypeptide, the said polypeptide being **characterized in that** it has the following amino acid sequence SEQ ID NO: 1:

Met Gly Ser Ser Arg Ala Pro Trp Met Gly Arg Val Gly Gly His Gly
1               5                   10                  15
Met Met Ala Leu Leu Leu Ala Gly Leu Leu Leu Pro Gly Thr Leu Ala
                20                  25                  30
Lys Ser Ile Gly Thr Phe Ser Asp Pro Cys Lys Asp Pro Thr Arg Ile
            35                  40                  45
Thr Ser Pro Asn Asp Pro Cys Leu Thr Gly Lys Gly Asp Ser Ser Gly
    50                  55                  60
Phe Ser Ser Tyr Ser Gly Ser Ser Ser Ser Gly Ser Ser Ile Ser Ser
65                  70                  75                  80
Ala Arg Ser Ser Gly Gly Gly Ser Ser Gly Ser Ser Ser Gly Ser Ser
                85                  90                  95
Ile Ala Gln Gly Gly Ser Ala Gly Ser Phe Lys Pro Gly Thr Gly Tyr
            100                 105                 110
Ser Gln Val Ser Tyr Ser Ser Gly Ser Gly Ser Ser Leu Gln Gly Ala
        115                 120                 125
Ser Gly Ser Ser Gln Leu Gly Ser Ser Ser Ser His Ser Gly Ser Ser
    130                 135                 140
Gly Ser His Ser Gly Ser Ser Ser Ser His Ser Ser Ser Ser Ser Ser
145                 150                 155                 160
Phe Gln Phe Ser Ser Ser Ser Phe Gln Val Gly Asn Gly Ser Ala Leu
                165                 170                 175
Pro Thr Asn Asp Asn Ser Tyr Arg Gly Ile Leu Asn Pro Ser Gln Pro
            180                 185                 190
Gly Gln Ser Ser Ser Ser Ser Gln Thr Ser Gly Val Ser Ser Ser Gly
        195                 200                 205
Gln Ser Val Ser Ser Asn Gln Arg Pro Cys Ser Ser Asp Ile Pro Asp
    210                 215                 220
Ser Pro Cys Ser Gly Gly Pro Ile Val Ser His Ser Gly Pro Tyr Ile
225                 230                 235                 240
Pro Ser Ser His Ser Val Ser Gly Gly Gln Arg Pro Val Val Val Val

                245                 250                 255
Val Asp Gln His Gly Ser Gly Ala Pro Gly Val Val Gln Gly Pro Pro
            260                 265                 270
Cys Ser Asn Gly Gly Leu Pro Gly Lys Pro Cys Pro Pro Ile Thr Ser
        275                 280                 285
Val Asp Lys Ser Tyr Gly Gly Tyr Glu Val Val Gly Gly Ser Ser Asp
    290                 295                 300
Ser Tyr Leu Val Pro Gly Met Thr Tyr Ser Lys Gly Lys Ile Tyr Pro
305                 310                 315                 320
Val Gly Tyr Phe Thr Lys Glu Asn Pro Val Lys Gly Ser Pro Gly Val
                325                 330                 335
Pro Ser Phe Ala Ala Gly Pro Pro Ile Ser Glu Gly Lys Tyr Phe Ser
            340                 345                 350
Ser Asn Pro Ile Ile Pro Ser Gln Ser Ala Ala Ser Ser Ala Ile Ala
        355                 360                 365
Phe Gln Pro Val Gly Thr Gly Gly Val Gln Leu Cys Gly Gly Gly Ser
    370                 375                 380
Thr Gly Ser Lys Gly Pro Cys Ser Pro Ser Ser Ser Arg Val Pro Ser
385                 390                 395                 400
Ser Ser Ser Ile Ser Ser Ser Ala Gly Ser Pro Tyr His Pro Cys Gly
                405                 410                 415
Ser Ala Ser Gln Ser Pro Cys Ser Pro Pro Gly Thr Gly Ser Phe Ser
            420                 425                 430
Ser Ser Ser Ser Ser Gln Ser Ser Gly Lys Ile Ile Leu Gln Pro Cys
        435                 440                 445
Gly Ser Lys Ser Ser Ser Ser Gly His Pro Cys Met Ser Val Ser Ser
    450                 455                 460
Leu Thr Leu Thr Gly Gly Pro Asp Gly Ser Pro His Pro Asp Pro Ser
465                 470                 475                 480
Ala Gly Ala Lys Pro Cys Gly Ser Ser Ser Ala Gly Lys Ile Pro Cys
                485                 490                 495
Arg Ser Ile Arg Asp Ile Leu Ala Gln Val Lys Pro Leu Gly Pro Gln
            500                 505                 510
Leu Ala Asp Pro Glu Val Phe Leu Pro Gln Gly Glu Leu Leu Asp Ser
        515                 520                 525
Pro

2. Composition according to Claim 1, **characterized in that** the polypeptide is purified from mammals.

3. Composition according to any one of the preceding claims, **characterized in that** the polypeptide is purified from mammalian skin.

4. Composition according to any one of the preceding claims, **characterized in that** the polypeptide is purified from human skin.

5. Composition according to any one of the preceding claims, **characterized in that** the polypeptide is purified from human epidermis.

6. Composition according to any one of the preceding claims, **characterized in that** the polypeptide is involved in intercorneocyte cohesion.

7. Composition according to any one of the preceding claims, **characterized in that** the polypeptide partly consists of the polypeptide sequence as described in Claim 1.

8. Composition according to any one of the preceding claims, **characterized in that** the polypeptide has an apparent molecular weight, as can be determined by SDS-polyacrylamide gel electrophoresis (SDS-PAGE), of between 50 and 60 kilodaltons, preferably between 52 and 56 kilodaltons.

9. Composition according to any one of the preceding claims, **characterized in that** the polypeptide is basic.

10. Composition according to any one of the preceding claims, **characterized in that** the polypeptide is glycosylated.

11. Composition according to any one of the preceding claims, **characterized in that** the polypeptide is phosphorylated.

12. Composition according to any one of the preceding claims, **characterized in that** the polypeptide is phosphorylated, basic and glycosylated and **in that** the polypeptide has an apparent molecular weight, as can be determined by SDS-polyacrylamide gel electrophoresis (SDS-PAGE), of between 50 and 60 kilodaltons, preferably between 52 and 56 kilodaltons.

13. Composition according to any one of Claims 1 to 12 for treating the thinning of the epidermis, and in particular the horny layer, and/or for treating excessive fragility of the skin covering and/or for strengthening intercorneocyte cohesion and/or inducing the thickening of the horny layer.

14. Composition according to any one of Claims 1 to 12 for treating trophic skin disorders or those following cicatrization disorders.

15. Use of at least one polypetide having the sequence SEQ ID NO: 1 for the preparation of a cosmetic composition according to any one of Claims 1 to 12, the said composition being intended for the cosmetic treatment of the thinning of the epidermis, and in particular the horny layer, and/or for treating excessive fragility of the skin covering and/or for strengthening intercorneocyte cohesion and/or inducing the thickening of the horny layer, the said composition being suitable for application to the skin of the subject to be treated.

16. Use of at least one polypeptide having the sequence SEQ ID NO: 1 for the preparation of a cosmetic composition according to any one of Claims 1 to 12, the said composition being intended for the treatment of trophic skin disorders or those following cicatrization disorders, and being suitable for application to the skin of the subject to be treated.

17. Cosmetic or pharmaceutical composition comprising, in a physiologically acceptable medium, at least the following coding nucleotide sequence SEQ ID NO: 2:

1 ATG GGC TCG TCT CGG GCA CCC TGG ATG GGG CGT GTG GGT GGG CAC
46 GGG ATG ATG GCA CTG CTG CTG GCT GGT CTC CTC CTG CCA GGG ACC
91 TTG GCT AAG AGC ATT GGC ACC TTC TCA GAC CCC TGT AAG GAC CCC
136 ACG CGT ATC ACC TCC CCT AAC GAC CCC TGC CTC ACT GGG AAG GGT
181 GAC TCC AGC GGC TTC AGT AGC TAC AGT GGC TCC AGC AGT TCT GGC
226 AGC TCC ATT TCC AGT GCC AGA AGC TCT GGT GGT GGC TCC AGT GGT
271 AGC TCC AGC GGA TCC AGC ATT GCC CAG GGT GGT TCT GCA GGA TCT
316 TTT AAG CCA GGA ACG GGG TAT TCC CAG GTC AGC TAC TCC TCC GGA
361 TCT GGC TCT AGT CTA CAA GGT GCA TCC GGT TCC TCC CAG CTG GGG
406 AGC AGC AGC TCT CAC TCG GGA AGC AGC GGC TCT CAC TCG GGA AGC
451 AGC AGC TCT CAT TCG AGC AGC AGC AGC AGC TTT CAG TTC AGC AGC
496 AGC AGC TTC CAA GTA GGG AAT GGC TCT GCT CTG CCA ACC AAT GAC
541 AAC TCT TAC CGC GGA ATA CTA AAC CCT TCC CAG CCT GGA CAA AGC
586 TCT TCC TCT TCC CAA ACC TCT GGG GTA TCC AGC AGT GGC CAA AGC
631 GTC AGC TCC AAC CAG CGT CCC TGT AGT TCG GAC ATC CCC GAC TCT
676 CCC TGC AGT GGA GGG CCC ATC GTC TCG CAC TCT GGC CCC TAC ATC
721 CCC AGC TCC CAC TCT GTG TCA GGG GGT CAG AGG CCT GTG GTG GTG
766 GTG GTG GAC CAG CAC GGT TCT GGT GCC CCT GGA GTG GTT CAA GGT
811 CCC CCC TGT AGC AAT GGT GGC CTT CCA GGC AAG CCC TGT CCC CCA
856 ATC ACC TCT GTA GAC AAA TCC TAT GGT GGC TAC GAG GTG GTG GGT
901 GGC TCC TCT GAC AGT TAT CTG GTT CCA GGC ATG ACC TAC AGT AAG
946 GGT AAA ATC TAT CCT GTG GGC TAC TTC ACC AAA GAG AAC CCT GTG
991 AAA GGC TCT CCA GGG GTC CCT TCC TTT GCA GCT GGG CCC CCC ATC
1036 TCT GAG GGC AAA TAC TTC TCC AGC AAC CCC ATC ATC CCC AGC CAG
1081 TCG GCA GCT TCC TCG GCC ATT GCG TTC CAG CCA GTG GGG ACT GGT
1126 GGG GTC CAG CTC TGT GGA GGC GGC TCC ACG GGC TCC AAG GGA CCC
1171 TGC TCT CCC TCC AGT TCT CGA GTC CCC AGC AGT TCT AGC ATT TCC
1216 AGC AGC GCC GGT TCA CCC TAC CAT CCC TGC GGC AGT GCT TCC CAG
1261 AGC CCC TGC TCC CCA CCA GGC ACC GGC TCC TTC AGC AGC AGC TCC
1306 AGT TCC CAA TCG AGT GGC AAA ATC ATC CTT CAG CCT TGT GGC AGC
1351 AAG TCC AGC TCT TCT GGT CAC CCT TGC ATG TCT GTC TCC TCC TTG
1396 ACA CTG ACT GGG GGC CCC GAT GGC TCT CCC CAT CCT GAT CCC TCC
1441 GCT GGT GCC AAG CCC TGT GGC TCC AGC AGT GCT GGA AAG ATC CCC
1486 TGC CGC TCC ATC CGG GAT ATC CTA GCC CAA GTG AAG CCT CTG GGG
1531 CCC CAG CTA GCT GAC CCT GAA GTT TTC CTA CCC CAA GGA GAG TTA
1576 CTC GAC AGT CCA TAA

## Patentansprüche

1. Kosmetische oder pharmazeutische Zusammensetzung, die in einem physiologisch akzeptablen Medium mindestens ein gereinigtes natürliches oder synthetisches Polypeptid enthält, wobei das Polypeptid **dadurch gekennzeichnet ist, dass** es die folgende Aminosäuresequenz SEQ ID NO: 1 aufweist:

Met Gly Ser Ser Arg Ala Pro Trp Met Gly Arg Val Gly Gly His Gly
1               5                   10                  15
Met Met Ala Leu Leu Leu Ala Gly Leu Leu Leu Pro Gly Thr Leu Ala
            20                  25                  30
Lys Ser Ile Gly Thr Phe Ser Asp Pro Cys Lys Asp Pro Thr Arg Ile
        35                  40                  45
Thr Ser Pro Asn Asp Pro Cys Leu Thr Gly Lys Gly Asp Ser Ser Gly
    50                  55                  60
Phe Ser Ser Tyr Ser Gly Ser Ser Ser Gly Ser Ser Ile Ser Ser
65 70 75 80
Ala Arg Ser Ser Gly Gly Gly Ser Ser Gly Ser Ser Ser Gly Ser Ser
85 90 95
Ile Ala Gln Gly Gly Ser Ala Gly Ser Phe Lys Pro Gly Thr Gly Tyr
                100                 105                 110
Ser Gln Val Ser Tyr Ser Ser Gly Ser Gly Ser Ser Leu Gln Gly Ala
            115                 120                 125
Ser Gly Ser Ser Gln Leu Gly Ser Ser Ser Ser His Ser Gly Ser Ser
        130                 135                 140
Gly Ser His Ser Gly Ser Ser Ser Ser His Ser Ser Ser Ser Ser Ser
145 150 155 160
Phe Gln Phe Ser Ser Ser Ser Phe Gln Val Gly Asn Gly Ser Ala Leu
165 170 175
Pro Thr Asn Asp Asn Ser Tyr Arg Gly Ile Leu Asn Pro Ser Gln Pro
                180                 185                 190
Gly Gln Ser Ser Ser Ser Ser Gln Thr Ser Gly Val Ser Ser Ser Gly
            195                 200                 205
Gln Ser Val Ser Ser Asn Gln Arg Pro Cys Ser Ser Asp Ile Pro Asp
        210                 215                 220

Ser Pro Cys Ser Gly Gly Pro Ile Val Ser His Ser Gly Pro Tyr Ile
225 230 235 240
Pro Ser Ser His Ser Val Ser Gly Gly Gln Arg Pro Val Val Val Val
245 250 255
Val Asp Gln His Gly Ser Gly Ala Pro Gly Val Val Gln Gly Pro Pro
                260                 265                 270
Cys Ser Asn Gly Gly Leu Pro Gly Lys Pro Cys Pro Pro Ile Thr Ser
            275                 280                 285
Val Asp Lys Ser Tyr Gly Gly Tyr Glu Val Val Gly Gly Ser Ser Asp
        290                 295                 300
Ser Tyr Leu Val Pro Gly Met Thr Tyr Ser Lys Gly Lys Ile Tyr Pro
305 310 315 320
Val Gly Tyr Phe Thr Lys Glu Asn Pro Val Lys Gly Ser Pro Gly Val
325 330 335
Pro Ser Phe Ala Ala Gly Pro Pro Ile Ser Glu Gly Lys Tyr Phe Ser
                340                 345                 350
Ser Asn Pro Ile Ile Pro Ser Gln Ser Ala Ala Ser Ser Ala Ile Ala
            355                 360                 365
Phe Gln Pro Val Gly Thr Gly Gly Val Gln Leu Cys Gly Gly Gly Ser
        370                 375                 380
Thr Gly Ser Lys Gly Pro Cys Ser Pro Ser Ser Ser Arg Val Pro Ser
385 390 395 400
Ser Ser Ser Ile Ser Ser Ser Ala Gly Ser Pro Tyr His Pro Cys Gly
405 410 415
Ser Ala Ser Gln Ser Pro Cys Ser Pro Pro Gly Thr Gly Ser Phe Ser
                420                 425                 430
Ser Ser Ser Ser Ser Gln Ser Ser Gly Lys Ile Ile Leu Gln Pro Cys
            435                 440                 445
Gly Ser Lys Ser Ser Ser Ser Gly His Pro Cys Met Ser Val Ser Ser
        450                 455                 460
Leu Thr Leu Thr Gly Gly Pro Asp Gly Ser Pro His Pro Asp Pro Ser
465 470 475 480
Ala Gly Ala Lys Pro Cys Gly Ser Ser Ser Ala Gly Lys Ile Pro Cys
485 490 495
Arg Ser Ile Arg Asp Ile Leu Ala Gln Val Lys Pro Leu Gly Pro Gln
                500                 505                 510
Leu Ala Asp Pro Glu Val Phe Leu Pro Gln Gly Glu Leu Leu Asp Ser
            515                 520                 525
Pro

2. Zusammensetzung nach Anspruch 1, **dadurch gekennzeichnet, dass** das Polypeptid aus Säugern gereinigt ist.

3. Zusammensetzung nach einem der vorhergehenden Ansprüche, **dadurch gekennzeichnet, dass** das Polypeptid aus Haut von Säugern gereinigt ist.

4. Zusammensetzung nach einem der vorhergehenden Ansprüche, **dadurch gekennzeichnet, dass** das Polypeptid aus menschlicher Haut gereinigt ist.

5. Zusammensetzung nach einem der vorhergehenden Ansprüche, **dadurch gekennzeichnet, dass** das Polypeptid aus menschlicher Epidermis gereinigt ist.

6. Zusammensetzung nach einem der vorhergehenden Ansprüche, **dadurch gekennzeichnet, dass** das Polypeptid an der interkorneozytären Kohäsion beteiligt ist.

7. Zusammensetzung nach einem der vorhergehenden Ansprüche, **dadurch gekennzeichnet, dass** das Polypeptid zum Teil aus der Sequenz des Polypeptids besteht, das in Anspruch 1 beschrieben ist.

8. Zusammensetzung nach einem der vorhergehenden Ansprüche, **dadurch gekennzeichnet, dass** das Polypeptid ein apparentes Molekulargewicht aufweist, wie es durch SDS-Polyacrylamid-Gelelektrophorese (SDS-PAGE) bestimmt werden kann, das im Bereich von 50 bis 60 Kilodalton, vorzugsweise im Bereich von 52 bis 56 Kilodalton, liegt.

9. Zusammensetzung nach einem der vorhergehenden Ansprüche, **dadurch gekennzeichnet, dass** das Polypeptid basisch ist.

10. Zusammensetzung nach einem der vorhergehenden Ansprüche, **dadurch gekennzeichnet, dass** das Polypeptid glykosyliert ist.

11. Zusammensetzung nach einem der vorhergehenden Ansprüche, **dadurch gekennzeichnet, dass** das Polypeptid phosphoryliert ist.

12. Zusammensetzung nach einem der vorhergehenden Ansprüche, **dadurch gekennzeichnet, dass** das Polypeptid phosphoryliert, basisch, glykosyliert ist und dass das Polypeptid ein apparentes Molekulargewicht aufweist, wie es durch SDS-Polyacrylamid-Gelelektrophorese (SDS-PAGE) bestimmt werden kann, das im Bereich von 50 bis 60 Kilodalton, vorzugsweise im Bereich von 52 bis 56 Kilodalton, liegt.

13. Zusammensetzung nach einem der Ansprüche 1 bis 12, die zum Behandeln des Dünnerwerdens der Epidermis und insbesondere der Hornschicht und/oder zum Behandeln einer übermäßigen Empfindlichkeit des Hautüberzugs und/ oder zum Verstärken der interkorneozytären Kohäsion und/oder zum Auslösen des Dickerwerdens der Hornschicht vorgesehen ist.

14. Zusammensetzung nach einem der Ansprüche 1 bis 12, die zum Behandeln der trophischen Hautstörungen oder zum Behandeln der Störungen, die eine Folge der Störungen der Wundheilung sind, vorgesehen ist.

15. Verwendung mindestens eines Polypeptids mit der Sequenz SEQ ID NO: 1 für die Herstellung einer kosmetischen Zusammensetzung nach einem der Ansprüche 1 bis 12, wobei die Zusammensetzung für die kosmetische Behandlung des Dünnerwerdens der Epidermis und insbesondere der Hornschicht und/oder zum Behandeln einer übermäßigen Empfindlichkeit des Hautüberzugs und/ oder zum Verstärken der interkorneozytären Kohäsion und/ oder zum Auslösen des Dickerwerdens der Hornschicht vorgesehen ist, wobei die Zusammensetzung für eine Anwendung auf der Haut der zu behandelnden Person geeignet ist.

16. Verwendung mindestens eines Polypeptids mit der Sequenz SEQ ID NO: 1 für die Herstellung einer kosmetischen Zusammensetzung nach einem der Ansprüche 1 bis 12, wobei die Zusammensetzung für die Behandlung der trophischen Hautstörungen oder die Behandlung der Störungen, die eine Folge der Störungen der Wundheilung sind, vorgesehen ist und wobei die Zusammensetzung für eine Anwendung auf der Haut der zu behandelnden Person geeignet ist.

17. Kosmetische oder pharmazeutische Zusammensetzung, die in einem physiologisch akzeptablen Medium mindestens die folgende codierende Nucleotidsequenz SEQ ID NO: 2 enthält:
